# FASCICULE DE BREVET EUROPEEN

(11) **EP 1 882 464 B1**
(45) Date de publication et mention de la délivrance du brevet: **02.03.2011**
(21) Numéro de dépôt: 06121144.7
(22) Date de dépôt: 22.09.2006
(51) Int. Cl.: A61F 2/44

(54) **Implant intervertébral destiné à la fusion osseuse**
Zwischenwirbelimplantat zur Knochenverbindung
Intervertebral implant for bone fusion

(30) Priorité: 27.07.2006 FR 0653141
(43) Date de publication de la demande: 30.01.2008
(73) Titulaire: Creaspine, 33600 Pessac (FR)
(72) Inventeur: Bernard, Marc, 33850, LEOGNAN (FR); Jenny, Philippe, 33600, PESSAC (FR); Lehuec, Jean-Charles, 33600, PESSAC (FR)
(74) Mandataire: Gaillarde, Frédéric F. Ch.

(56) Documents cités:
- WO-A-99/47083
- WO-A-03/037229
- WO-A-2005/063151
- US-A1- 2004 102 850

## Description

La présente invention concerne un implant intervertébral destiné à la reconstruction d'un corps vertébral grâce à l'introduction des greffons osseux naturels ou synthétiques entre deux corps vertébraux pour remplacer une zone de la colonne vertébrale endommagée ou combler une zone de carence de la colonne vertébrale en permettant une croissance des cellules osseuses au travers de l'implant.

Le document US 2004/0102850 A représente l'état de la technique le plus proche et détermine le préambule de la revendication 1.

Ces implants doivent remplir plusieurs critères pour assurer une bonne stabilité entre deux corps vertébraux tout en permettant de garantir un taux de fusion satisfaisant. Pour cela le corps de l'implant qui est introduit sous pression entre les corps vertébraux doit posséder d'une part une très bonne résistance axiale selon la colonne vertébrale et d'autre part une surface de contact importante avec les corps vertébraux adjacents et les éléments résiduels.

L'implant doit avoir une résistance mécanique importante par rapport aux contraintes exercées par les corps vertébraux pour maintenir l'espace intervertébral. De manière générale dans la pratique l'implant doit posséder une résistance 2 à 3 fois supérieure aux forces exercées par les corps vertébraux sur l'implant. L'implant doit être résistant au moins jusqu'à la consolidation osseuse.

De manière générale, l'implant est un corps creux solide rempli de greffons osseux. Les parois sont ajourées pour permettre la fusion osseuse avec les corps vertébraux.

L'implant est placé entre deux corps vertébraux soit horizontalement dans le sens de son épaisseur soit verticalement dans le sens de sa hauteur.

L'implant peut être classifié selon la forme du corps. Elle peut être parallélépipédique de section rectangulaire. Pour mieux s'adapter à la forme des corps vertébraux, elle peut être ogivale ou légèrement arrondie.

On connaît l'implant constitué d'une cage cylindrique ou ovale creuse intérieurement pour recevoir les greffons osseux, la paroi de la cage comportant un ensemble d'ouvertures. Ce type d'implant permet une fusion osseuse autour de l'implant et avec les plateaux vertébraux, par contre le centre des greffons ne fusionne pas ou que partiellement.

Il existe également des implants comportant seulement deux plateaux disposés aux extrémités d'un axe. Le chirurgien est amené à remplir l'implant de greffons compactés tout autour de l'axe une fois que l'implant soit disposé entre deux corps vertébraux. Ce type d'implant offre effectivement une surface de contact circonférentielle relativement importante avec le milieu extérieur. Cependant par sa forme il ne permet pas de maintenir les greffons à l'emplacement de la zone nécessitant une reconstruction osseuse. Par ailleurs, l'insert des greffons dans l'implant allonge le temps opératoire.

Il serait donc intéressant de disposer d'un implant ayant une structure très résistante tout en ayant une surface de contact importante et en continu avec l'extérieur pour la fusion osseuse et une forme rétentrice optimale de façon à retenir les greffons osseux.

L'objectif de la présente invention est donc de proposer un implant intervertébral pour la fusion osseuse, simple dans sa conception et dans son mode opératoire, particulièrement compact et résistant pour permettre une meilleure fusion entre les greffons osseux et les plateaux vertébraux dans toutes les directions autour de l'implant.

Cet implant peut en particulier être rempli partiellement de greffons osseux par le chirurgien avant l'insertion de l'implant dans le corps du patient, ledit implant, qui, une fois inséré dans la zone à réparer, est entièrement rempli par le chirurgien. La longueur de l'implant est ajustable par le fait qu'il est possible soit de le couper soit d'assembler plusieurs implants bout à bout ensemble et le rend avantageusement compatible avec une zone de carence de longueur importante.

En outre, l'implant doit posséder par sa forme un axe solide permettant une reprise des contraintes exercées par les corps vertébraux adjacents.

Par ailleurs, par sa forme relativement compacte, le procédé de fabrication de l'implant est très simple à mettre oeuvre.

A cet effet, l'invention concerne un implant pour la fusion osseuse destiné à être inséré entre deux corps vertébraux adjacents comme défini dans la revendication 1.

Selon l'invention, ledit implant comporte à ses extrémités une surface d'appui destinée à venir en contact avec les corps vertébraux.

Dans une forme particulièrement avantageuse de réalisation de l'invention, ladite surface d'appui est constituée directement par les extrémités desdites élément.

Dans une autre forme de réalisation de l'invention, ladite surface d'appui est constituée par deux plateaux, chaque plateau comprenant au moins un orifice placé en regard des extrémités d'au moins deux évidements longitudinaux.

Dans différents modes de réalisation particuliers de cet implant intervertébral, chacun ayant ses avantages particuliers et susceptibles de nombreuses combinaisons techniques possibles :
- lesdits au moins deux éléments de profil en C et lesdits plateaux forment une pièce monobloc.
- lesdits au moins deux éléments de profil en C et lesdits plateaux ne forment pas une pièce monobloc, lesdits plateaux comportant chacun une face d'accouplement apte à venir coopérer avec l'extrémité desdits éléments et une face d'appui destinée à venir au contact du corps vertébral

Avantageusement, les extrémités desdits éléments et les faces d'appui desdits plateaux comporte un ensemble de saillies destinées à renforcer la prise d'appui contre le corps vertébral.

Les éléments ont une dimension longitudinale comprise entre environ 16 mm et 90 mm et un diamètre compris entre environ 10 et 36 mm.

Dans un mode de réalisation particulier de l'invention, l'implant comporte en outre à ses extrémités une surface d'accouplement apte à venir coopérer avec un élément de jonction, ledit élément de jonction étant destiné à assembler par deux, des corps principaux ensemble.

Avantageusement ledit axe solide de l'implant comporte au moins une ouverture de manière à créer une communication entre les deux évidements longitudinaux.

L'implant est réalisé dans un matériau biocompatible, ledit corps principal étant réalisé avantageusement dans un matériau radiotransparent et lesdits plateaux dans un matériau radiovisible.

Selon une forme avantageuse de réalisation de l'invention, l'implant comporte des moyens de positionnements réalisés dans la surface d'appui, lesdits moyens coopérant avec l'extrémité de chaque bras d'un distracteur, la dimension desdits moyens étant adaptée à la dimension de l'extrémité du bras de sorte que ladite extrémité puisse coulisser lors du retrait dudit distracteur, et que la surface d'appui dudit implant soient en contact direct avec les corps vertébraux adjacents maintenus écartés par lesdits bras lors de la mise en place de l'implant.

Selon l'invention, lesdits moyens sont constitués par une rainure.

L'invention concerne également un procédé pour réaliser un implant décrit ci-dessus constitué d'au moins deux éléments en forme C.

L'invention sera décrite plus en détail en référence aux dessins annexés dans lesquels :
- la figure 1 représente une vue en biais d'un implant selon l'invention;
- la figure 2.A représente une forme de réalisation particulière de l'implant de la figure 1 comportant des plateaux à ses extrémités et la figure 2.B représente une vue éclatée de l'assemblage entre les plateaux et le corps principal ;
- la figure 3 représente une autre forme particulière de l'implant de la figure 1 ;
- la figure 4 est une représentation schématique de l'implant de la figure 1 inséré entre deux corps vertébraux,
- la figure 5 est une représentation schématique de l'implant de la figure 1 comportant une rainure à ses extrémités,
- la figure 6 est une représentation schématique de l'implant de la figure 2 comportant une rainure dans les plateaux.

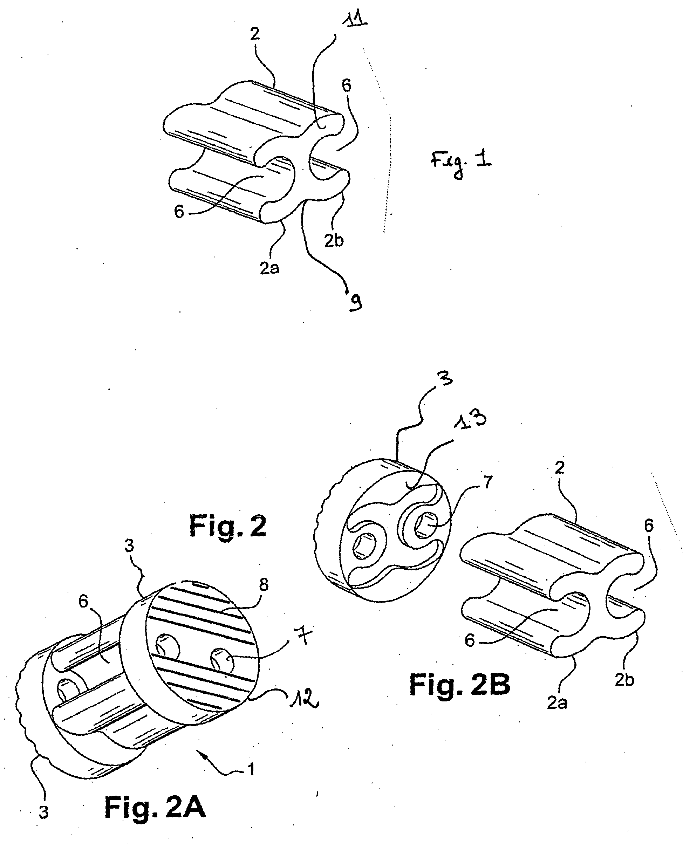

La Figure 1 représente un implant 1 selon l'invention. Il comporte un corps principal 2 ayant un axe principal. Lors du positionnement de cet implant entre deux corps vertébraux, cet axe principal est orienté généralement parallèlement à l'axe de la colonne vertébrale. Il comporte un corps principal constitué de deux éléments longitudinaux de profil en C 2a, 2b, placés dos à dos, l'ensemble délimitant deux évidements longitudinaux 6 destinés à retenir des greffons. Par cette forme particulièrement avantageuse, l'implant comporte un axe solide 9 constitué par les parois desdits éléments en contact. Cet axe solide permet ainsi une reprise efficace des contraintes exercées par les corps vertébraux sur l'implant.

Les extrémités 11 des éléments 2a, 2b constituent directement des surfaces d'appui pour l'implant destinées à venir en contact des corps vertébraux adjacents 4.

Par ailleurs, chaque évidement 6 comporte une ouverture longitudinale orientée vers l'extérieur par rapport à l'axe solide 9, permettant ainsi aux greffons d'être en contact de manière circonférentielle et en continu avec le milieu extérieur.

En outre, cette forme particulièrement avantageuse de l'implant permet d'obtenir le corps principal par un procédé plus simple et moins coûteux par rapport aux implants de forme classique. Le procédé pour fabriquer un tel implant est un procédé d'extrusion dans lequel on réalise un filage d'un matériau plastique à chaud. On forme le matériau à travers une filière ayant une forme complémentaire de celle de l'implant pour former une pièce en continu. Les implants de différentes longueurs sont ensuite obtenus en réalisant des découpes dans la pièce en continu. L'implant de la figure 1 ne nécessite pas d'étape supplémentaire d'usinage ou de autre procédé.

La figure 2.A montre une autre forme de réalisation de l'implant dans laquelle la surface d'appui de l'implant est constituée par deux plateaux 3 situés aux extrémités du corps principal 2 et la surface d'appui est l'une des deux faces 12 du plateau.

Afin de favoriser la fusion osseuse avec les corps vertébraux adjacents, la figure 2.A montre que les plateaux 3 comportent des orifices 7 placé en regard des extrémités des évidements longitudinaux 6 afin d'avoir une surface de contact entre les greffons retenus dans les évidements longitudinaux et les corps vertébraux adjacents pour favoriser la fusion osseuse dans une direction verticale.

Les plateaux 3 et les éléments 2a, 2b peuvent être formés à partir d'une seule pièce monobloc ou formés à partir d'éléments distincts.

La figure 2.B montre un assemblage entre un des deux plateaux et le corps principal 2. Le plateau 3 comporte une face d'accouplement 7 apte à venir à coopérer avec l'extrémité du corps principal 2 formée par les deux éléments 2a, 2b et une face d'appui 12 destinée à venir au contact du corps vertébral.

Le plateau 3 et l'extrémité du corps principal 2 sont ainsi avantageusement reliés de manière amovible l'un à l'autre pour permettre de varier la longueur de l'implant et à adapter celui-ci à la zone de la colonne vertébrale à réparer. Ainsi les corps principaux peuvent être mis bout à bout et assemblés ensemble par des éléments de jonction. Dans ce cadre, chaque corps principal est relié à un seul corps pour ceux qui sont destinés aux extrémité de l'implant, ou deux autres par un élément de jonction monté de part et d'autre des extrémités du corps principal. Chaque élément de jonction comporte deux faces d'accouplement permettant de recevoir chacune l'extrémité du corps principal.

Ces faces d'accouplement ont un profil complémentaire du profil du corps principal mais peuvent avoir tout autre forme permettant de bloquer les corps principaux lorsqu'ils sont mis bout à bout.

Ainsi le nombre de corps principal constituant un implant n'est pas limitatif dans les deux formes de réalisations décrites ci-dessus.

Il est possible par exemple d'obtenir un implant de longueur de 64 mm en mettant bout à bout quatre corps principaux ensemble ayant chacun une longueur de 16 mm.

Les éléments de profil en C 2 étant identiques ou non, ils ont une dimension longitudinale comprise de préférence entre environ 16 mm et 90 mm et un diamètre compris entre 10 et 36 mm.

Avantageusement le nombre d'éléments de profil en C pour former un corps principal n'est pas limitatif.

Avantageusement le profil de l'implant ainsi obtenu respecte la courbure anatomique de la colonne vertébrale.

Chaque élément de profil en C peut de plus comporter des organes mécaniques de verrouillage permettant de bloquer les faces d'accouplement des éléments de jonction en position accouplée. Ces organes de verrouillage sont par exemple des vis.

Avantageusement, les extrémités 11 des éléments 2a, 2b et les faces d'appui 12 des plateaux 3 comportent un ensemble de saillies 8 destinées à renforcer la prise d'appui contre le corps vertébral et à éviter le déplacement transversal de l'implant dans l'espace intervertébrale. Ces saillies 8 sont constituées par exemple par des crans ou un ensemble d'aspérités.

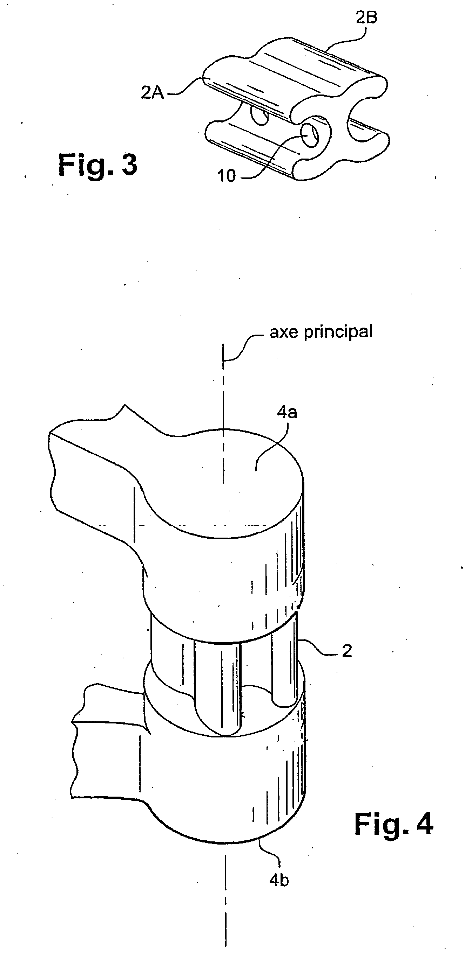

La figure 3 montre une autre forme de réalisation du corps principal 2 dans lequel des ouvertures 10 sont pratiquées au niveau de l'axe solide constituée par les parois externes des éléments de profil en C 2a, 2b en contact de manière à créer une communication entre les deux évidements longitudinaux. Cette communication entre les évidements favorise la qualité de la greffe et permet d'obtenir un seul et unique bloc de fusion.

Pour insérer l'implant entre deux corps vertébraux adjacents, on écarte tout d'abord deux corps vertébraux adjacents au moyen d'un distracteur munie à une extrémité deux bras qui réalise une espace intervertébral, on vient ensuite positionner l'implant entre les deux bras dans le sens de la hauteur du corps principal dans cette espace. Puis on retire les bras de manière à ne pas faire déplacer l'implant.

La figure 4 montre schématiquement un implant 1 réalisé selon l'invention positionné entre deux corps vertébraux. Pour augmenter la stabilité et la rigidité du montage, on effectue une ostéosynthèse postérieure et/ou antérieure par vis entre le corps vertébral sus-jacent 4a et le corps vertébral sous-jacent 4b par rapport à l'implant 1.

Dans une autre forme particulièrement avantageuse de l'invention, l'implant comporte une rainure 14 réalisée dans la surface d'appui 9, 12. Cette rainure coopérant avec l'extrémité de chaque bras du distracteur, permet d'une part de placer l'implant entre deux corps vertébraux en mettant la surface d'appui 9, 12 de l'implant en contact direct avec les corps vertébraux adjacents évitant ainsi une surdistraction des corps vertébraux et d'autre part un retrait efficace des extrémités du bras lors du retrait dudit distracteur, de manière à ne pas déplacer l'implant initialement positionné.

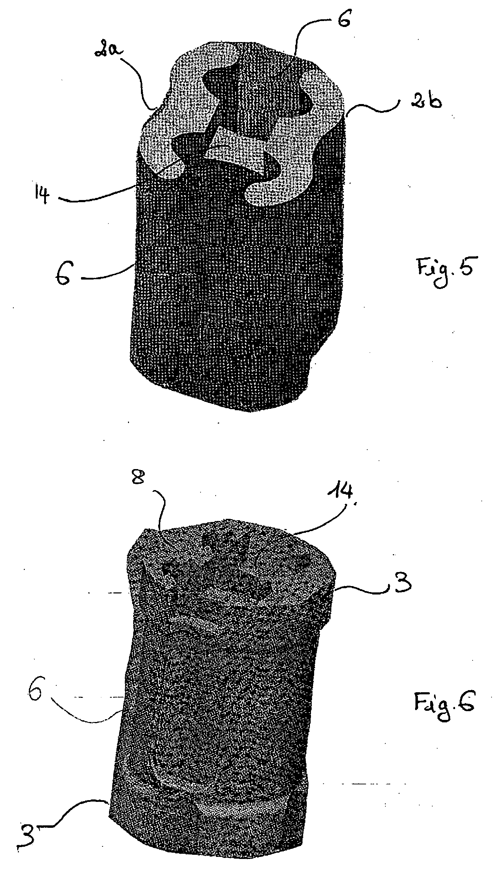

La figure 5 représente le cas où la rainure est réalisée directement dans les extrémités des éléments de profil en C 2a, 2b.

La figure 6 représente le cas où la rainure est réalisée dans les plateaux 3.

Avantageusement l'implant peut être partiellement rempli avant le positionnement de l'implant dans l'espace intervertébral. Mais les évidements longitudinaux peuvent être entièrement remplis permettant une réduction du temps opératoire. En outre l'effet de rétention des greffons au sein des évidements longitudinaux des éléments facilite l'opération de mise en place des greffons et rend plus précise le positionnement des greffons dans la zone à réparer. Cet effet de rétention résulte de la forme du profil des éléments en C.

Avantageusement la forme particulière de l'implant permet de répartir les contraintes exercées par les corps vertébraux sur les parois des éléments, et sur l'axe solide 9 constitué par les parois des éléments 2a, 2b qui se joignent. L'efficacité de cette reprise des contraintes peut être augmentée en ajoutant une surépaisseur au niveau l'axe solide 9.

L'implant est réalisé dans un matériau biocompatible. Le corps principal 2 est de préférence réalisé dans un matériau radiotransparent permettant ainsi de suivre l'évolution de la fusion osseuse et la consolidation osseuse chez le patient. Le matériau utilisé est généralement en polymère, par exemple un matériau de la famille de polyaryletheretherketone (PEEK). Le module d'élasticité du matériau est de préférence proche de l'os afin d'éviter le phénomène de déviation dû aux contraintes. Les plateaux 3 sont de préférence réalisés dans un matériau radiovisible de manière à pouvoir repérer l'emplacement de l'implant par une image radiologique.

## Revendications

1. Implant (1) pour la fusion osseuse destiné à être inséré entre deux corps vertébraux adjacents (4) d'une colonne vertébrale, l'implant comportant un corps principal (2) constitué d'au moins deux éléments (2a, 2b) ayant des parois placés dos à dos, les éléments définissant ainsi au moins deux évidements orientés vers l'extérieur de l'implant, **caractérisé en ce que** les éléments sont longitudinaux à profil en C, chaque évidement étant adapté pour retenir des greffons et ayant une ouverture longitudinale, l'implant ayant un axe solide constitué par la zone de jonction des parois desdits éléments en contact adapté pour être positionné parallèlement à l'axe de la colonne vertébrale et pour transmettre les efforts entre les deux corps vertébraux.

2. Implant selon la revendication 1, **caractérisé en ce qu'**il comporte à chacune de ses extrémités une surface d'appui destinée à venir en contact 15 respectivement avec l'un desdits corps vertébraux (4).

3. Implant selon la revendication 2, **caractérisé en ce que** lesdites surfaces d'appui sont constituées par les extrémités (11) desdits éléments (2a, 2b).

4. Implant selon la revendication 2, **caractérisé en ce que** lesdites 20 surfaces d'appui sont constituées par deux plateaux (3), chaque plateau comprenant au moins un orifice (7) débouchant dans l'un des évidements longitudinaux (6).

5. Implant selon la revendication 4, **caractérisé en ce que** lesdits éléments (2a, 2b) et lesdits plateaux (3) forment une pièce monobloc.

6. Implant selon la revendication 4, **caractérisé en ce que** lesdits éléments (2a, 2b) et lesdits plateaux (3) forment des pièces distinctes, lesdits plateaux comportant chacun une face d'accouplement (13) apte à venir coopérer avec une extrémité desdits éléments et une face d'appui (12) apte à venir au contact de l'un desdits corps vertébraux (4).

7. Implant selon l'une quelconque des revendications précédentes, **caractérisé en ce que** les extrémités desdits éléments et/ou lesdites surfaces d'appui (12) comportent des saillies (8) destinées à renforcer la prise d'appui contre les corps vertébraux (4).

8. Implant selon l'une quelconque des revendications précédentes, **caractérisé en ce que** lesdits éléments (2a, 2b) ont une dimension longitudinale comprise entre environ 16 mm et 90 mm et un diamètre compris entre environ 10 et 36 mm.

9. Implant selon l'une quelconque des revendications précédentes, **caractérisé en ce qu'**il comporte à au moins une de ses extrémités une surface d'accouplement apte à venir coopérer avec un élément de jonction, ledit élément de jonction étant adapté pour permettre l'assemblage d'implants entre eux.

10. Implant selon l'une quelconque des revendications précédentes, **caractérisé en ce que** ledit axe solide (9) comporte au moins une ouverture (10) de manière à créer une communication entre les deux évidements longitudinaux (6).

11. Implant selon l'une des revendications précédentes, **caractérisé en ce qu'**il est réalisé dans un matériau biocompatible, ledit corps principal étant réalisé dans un matériau radiotransparent.

12. Implant selon les revendications 4 et 11, **caractérisé en ce que** lesdits plateaux sont réalisés dans un matériau radiovisible.

13. Implant selon l'une quelconque des revendications précédentes, **caractérisé en ce qu'**il comporte des moyens de positionnement réalisés aux extrémités desdits éléments (2a, 2b), ces moyens étant adaptés pour coopérer respectivement avec les bras d'un distracteur, la dimension desdits moyens de positionnement étant adaptée à la dimension de ces bras de sorte que ladite extrémité puisse coulisser lors du retrait dudit distracteur, et que les extrémités dudit implant soient en contact direct avec les corps vertébraux adjacents maintenus écartés par lesdits bras lors de la mise en place de l'implant.

14. Implant selon la revendication 13, **caractérisé en ce que** lesdits moyens de positionnement comprennent une rainure (14).

15. Procédé pour réaliser ledit corps principal (2) de l'implant selon la revendication 1, **caractérisé en ce qu'**il comprend les étapes suivantes :
- obtention par extrusion en continu d'une pièce à partir d'une filière ayant une forme complémentaire de celle dudit corps principal (2) constitué d'au moins deux éléments en forme de C (2a, 2b),
- découpe de ladite pièce pour obtenir des corps principaux (2) de différentes longueurs.

## Claims

1. An implant (1) for bone fusion intended to be inserted between two adjacent vertebral bodies (4) of a spinal cord, the implant including a main body (2) consisting of at least two elements (2a, 2b) having walls placed back-to-back, the elements thereby defining at least two recesses oriented towards the outside of the implant, **characterized in that** the elements are longitudinal with a C-profile, each recess being adapted for retaining the grafts and having a longitudinal aperture, the implant having a solid axis formed by the junction area of the walls of said elements in contact adapted so as to be positioned parallel to the axis of the spinal cord and for transmitting the forces between both vertebral bodies.

2. The implant according to claim 1, **characterized in that** it includes at each of its ends a supporting surface intended to come into contact with one of said vertebral bodies (4) respectively.

3. The implant according to claim 2, **characterized in that** said supporting surfaces are formed by the ends (11) of said elements (2a, 2b).

4. The implant according to claim 2, **characterized in that** said supporting surfaces are formed by two plates (3), each plate comprising at least one orifice (7) opening out into one of the longitudinal recesses (6).

5. The implant according to claim 4, **characterized in that** said elements (2a, 2b) and said plates (3) form a one-piece part.

6. The implant according to claim 4, **characterized in that** said elements (2a, 2b) and said plates (3) form distinct parts, said plates each including a coupling face (13) capable of coming and co-operating with an end of said elements and a supporting face (12) capable of coming into contact with one of said vertebral bodies (4).

7. The implant according to any of the preceding claims, **characterized in that** the ends of said elements and/or said supporting surfaces (12) include protrusions (8) intended to reinforce the bearing action against the vertebral bodies (4).

8. The implant according to any of the preceding claims, **characterized in that** said elements (2a, 2b) have a longitudinal dimension comprised between about 16 mm and 90 mm and a diameter comprised between about 10 and 36 mm.

9. The implant according to any of the preceding claims, **characterized in that** it includes at least at one of its ends, one coupling surface capable of coming and co-operating with a junction element, said junction element being adapted so as to allow assembling of implants together.

10. The implant according to any of the preceding claims, **characterized in that** said solid axis (9) includes at least one aperture (10) so as to create communication between both longitudinal recesses (6).

11. The implant according to one of the preceding claims, **characterized in that** it is made in a biocompatible material, said main body being made in a radio-transparent material.

12. The implant according to claims 4 and 11, **characterized in that** said plates are made in a radio-visible material.

13. The implant according to any of the preceding claims, **characterized in that** it includes positioning means made at the ends of said elements (2a, 2b), these means being adapted for respectively co-operating with the arms of a distractor, the dimension of said positioning means being adapted to the dimension of these arms so that said end may slide upon withdrawing said distractor and that the ends of said implants are in direct contact with the adjacent vertebral bodies maintained separated by said arms during the setting into place of the implant.

14. The implant according to claim 13, **characterized in that** said positioning means comprise a groove (14).

15. A method for making said main body (2) of the implant according to claim 1, **characterized in that** it comprises the following steps:
- obtaining by continuous extrusion a part from a die having a complementary shape to that of said main body (2) consisting of at least two C-shaped elements (2a, 2b),
- cutting out said part in order to obtain main bodies (2) of different lengths.

## Patentansprüche

1. Implantat (1) für die Knochenfusion, das zum Einsetzen zwischen zwei benachbarten Wirbelkörpern (4) einer Wirbelsäule bestimmt ist, wobei das Implantat einen Hauptkörper (2) umfasst, der aus mindestens zwei Elementen (2a, 2b) besteht, die Rückseite an Rückseite platzierte Wände haben, wobei die Elemente **dadurch** mindestens zwei nach außerhalb des Implantats gerichtete Aussparungen definieren, **dadurch gekennzeichnet, dass** die Elemente länglich mit C-Profil sind, wobei jede Aussparung geeignet ist, Transplantate zurückzuhalten und eine längliche Öffnung hat, wobei das Implantat eine stabile Achse hat, die von dem Verbindungsbereich der Wände der Elemente im Kontakt gebildet wird, die geeignet ist, parallel zur Achse der Wirbelsäule positioniert zu sein und die Kräfte zwischen den zwei Wirbelkörpern zu übertragen.

2. Implantat nach Anspruch 1, **dadurch gekennzeichnet, dass** es an jedem seiner Enden eine Abstützfläche aufweist, die dazu bestimmt ist, mit jeweils einem der Wirbelkörper (4) in Kontakt zu kommen.

3. Implantat nach Anspruch 2, **dadurch gekennzeichnet, dass** die Abstützflächen von den Enden (11) der Elemente (2a, 2b) gebildet werden.

4. Implantat nach Anspruch 2, **dadurch gekennzeichnet, dass** die Abstützflächen von zwei Platten (3) gebildet werden, wobei jede Platte mindestens eine Öffnung (7) aufweist, die in eine der länglichen Aussparungen (6) mündet.

5. Implantat nach Anspruch 4, **dadurch gekennzeichnet, dass** die Elemente (2a, 2b) und die Platten (3) ein Einblockteil bilden.

6. Implantat nach Anspruch 4, **dadurch gekennzeichnet, dass** die Elemente (2a, 2b) und die Platten (3) voneinander verschiedene Teile bilden, wobei von den Platten jede jeweils eine Kopplungsfläche (13) umfasst, die imstande ist, mit einem Ende der Elemente zusammenzuarbeiten, und eine Abstützfläche (12), die imstande ist, mit einem der Wirbelkörper (4) in Kontakt zu kommen.

7. Implantat nach einem der vorangehenden Ansprüche, **dadurch gekennzeichnet, dass** die Ende der Elemente und/oder die Abstützflächen (12) Vorsprünge (8) aufweisen, die zur Verstärkung der Abstützung gegen die Wirbelkörper (4) bestimmt sind.

8. Implantat nach einem der vorangehenden Ansprüche, **dadurch gekennzeichnet, dass** die Elemente (2a, 2b) eine Abmessung längs zwischen zirka 16 mm und 90 mm inklusive und einen Durchmesser zwischen zirka 10 und 36 mm inklusive haben.

9. Implantat nach einem der vorangehenden Ansprüche, **dadurch gekennzeichnet, dass** es an mindestens einem seiner Enden eine Kupplungsfläche aufweist, die imstande ist, mit einem Verbindungselement zusammenzuarbeiten, wobei das Verbindungselement geeignet ist, die Verbindung von Implantaten untereinander zu erlauben.

10. Implantat nach einem der vorangehenden Ansprüche, **dadurch gekennzeichnet, dass** die stabile Achse (9) mindestens eine Öffnung (10) aufweist, um eine Verbindung zwischen den zwei länglichen Aussparungen (6) zu bilden.

11. Implantat nach einem der vorangehenden Ansprüche, **dadurch gekennzeichnet, dass** es aus einem biologisch kompatiblen Material hergestellt ist, wobei der Hauptkörper aus einem für Strahlen durchlässigen Material hergestellt ist.

12. Implantat nach den Ansprüchen 4 und 11, **dadurch gekennzeichnet, dass** die Platten aus einem für Strahlen sichtbaren Material hergestellt sind.

13. Implantat nach einem der vorangehenden Ansprüche, **dadurch gekennzeichnet, dass** es Positionierungsmittel an den Enden der Elemente (2a, 2b) aufweist, wobei diese Mittel geeignet sind, um jeweils mit den Armen eines Distraktors zusammenzuarbeiten, wobei die Abmessung der Positionierungsmittel an die Abmessung dieser Arme angepasst ist, damit das Ende beim Entfernen des Distraktors gleiten kann und die Enden des Implantat im direkten Kontakt mit den benachbarten Wirbelkörpern sind, die von den Armen beim Einsetzen des Implantats gespreizt gehalten werden.

14. Implantat nach Anspruch 13, **dadurch gekennzeichnet, dass** die Positionierungsmittel eine Rille (14) umfassen.

15. Verfahren zur Herstellung des Hauptkörpers (2) des Implantats nach Anspruch 1, **dadurch gekennzeichnet, dass** es die folgenden Schritte umfasst:
- Herstellen durch kontinuierliche Extrusion aus einer Düse eines Teils mit einer komplementären Form des Hauptkörpers (2), der aus mindestens zwei C-förmigen Elementen (2a, 2b) besteht,
- Schneiden des Teils, um Hauptkörper (2) unterschiedlicher Längen zu erhalten.
